# EUROPEAN PATENT SPECIFICATION

(11) **EP 2 964 101 B1**
(45) Date of publication and mention of the grant of the patent: **20.09.2017**
(21) Application number: 14710361.8
(22) Date of filing: 07.03.2014
(51) Int. Cl.: A61B 10/00, A61B 10/02

(54) **SAMPLE COLLECTION APPARATUS**
PROBENAHMEVORRICHTUNG
APPAREIL DE COLLECTE D'ÉCHANTILLON

(30) Priority: 07.03.2013 GB 201304117
(43) Date of publication of application: 13.01.2016
(73) Proprietor: Staffordshire University, Stafford ST18 0AD (GB)
(72) Inventor: BUURSTEDE, Jacobus Cornelis, Sprundel 4714 GV (NL); WALTON, Laura Marie, Stoke-on-Trent Staffordshire ST6 8JY (GB); PARTRIDGE, Julian Dennis, Newport Shropshire TF10 7DZ (GB); CASSELLA, John Paul, Swadlicote Derbyshire DE11 8NB (GB)
(74) Representative: Fennell, Gareth Charles
(86) International application number: PCT/GB2014/050691
(87) International publication number: WO 2014/135899

(56) References cited:
- WO-A1-97/32517
- US-A- 4 023 559
- US-A- 4 136 680
- US-A- 4 157 709
- US-A- 4 457 313
- US-A1- 2005 137 448
- US-A1- 2005 288 606

## Description

### Field

The present invention relates to a sample collection apparatus, in particular a sample collection apparatus for collecting samples from the high vaginal and endocervical region. The sample collection apparatus can be used for obtaining a sample during a sexual assault examination. The apparatus can also be used in the collection of vaginal material for medical purposes, such as cervical smears for cervical screening. The apparatus can also be used to collect samples from the anal cavity.

### Background

The current process of collecting samples from the high vaginal and endocervical region involves the insertion of the speculum or a proctoscope and is a very invasive process, which may cause discomfort. This problem applies for sexual assault examinations and the collection of vaginal material for medical purposes such as for cervical screening.

Another issue with the current process, in particular for sexual assault examinations, is the potential for contamination during the collection process. For example, as the speculum is inserted into the vagina, there is the potential for semen from the vulva or low vagina to be transferred to the high vagina or endocervical regions. This would result in a high vaginal swab producing a positive result when there was in fact no semen originally deposited in this region. The swabs currently in use have no protection to assure that the collected sample is only from the area stated in the Forensic Faculty of Legal Medicine (FFLM) regulation and has not been contaminated with fluids deriving from other areas. As the FFLM regulations indicate that the area where semen is found is important for the result interpretation and for the conclusion based on these results, it is important to be certain that the sample on the swab is not contaminated from introducing or retrieving the swab. The belief that contamination could have arisen would be sufficient to discount vital evidence, and could result in assailants being found not guilty when in fact the semen was present in the allotted region.

The present invention seeks to provide a new sample collection apparatus for collection of samples from the vaginal or anal cavities. The present invention also seeks to reduce discomfort during the examination process. The present invention also seeks to provide a collection apparatus which reduces the potential for contamination into specific areas, which may affect the accuracy of the interpretation of results.

US 2005/288606 A1 relates to a veterinary instrument for use in animal husbandry that includes an inner member with a swab or similar element attached at one end, located inside a second inner member meant to protect the innermost member and attached swab, and an outermost member that surrounds both inner members and has a closed mold manufactured protective cap on one end. The instrument has one or two safety pins which ensure that the instrument cannot be used until they are removed.

US 4 457 313 A relates to a shield protector device for use with artificial insemination instruments and/or culture collection devices, consisting of an inner tubular portion movable relative to an outer tubular sheath protector portion, includes a cylindrical housing having a closed end, which is sealingly attached to the distal end of the outer tubular sheath protector portion. The closed end of the housing is scored to provide a break-away membrane when the closed end of the shield protector is engaged by the inner tubular portion to complete the insemination or culture collection process.

US 4 136 680 A relates to a specimen collecting apparatus.

US 4 157 709 A relates to a probe for inserting a test element into the vaginal cavity while shielding it from intermediate vaginal contact, for positioning the test element precisely in contact with the cervical os in order to collect a specimen of cervical material therefrom, and for retrieving the test element and the specimen from the vaginal cavity while shielding them from intermediate vaginal contact. Processes are provided for retrieving specimens in the form of tissue or mucus.

US 2005/137448 A1 relates to an embryo transfer catheter with a protective sheath. The use of a protective sheath shields a guide catheter from cervical mucus. The retracted open flaps of the outer protective sheath draw the mucus and blood away from the inner guide catheter. This allows an inner embryo transfer sheath to enter the uterus unobstructed. The embryo is then transferred to the uterus by means of fluid pressure with a culture medium. The protective sheath may be snap fit onto the guide catheter. The sheaths and catheters may be manufactured in a variety of lengths for individual convenience and then snap-fit onto each other for better control during an embryo implantation procedure. Other catheters and related instruments may also be manufactured with snap-on features.

US 4 023 559 A relates to a sampling catheter comprising an outer tube of resilient material which has an open and a closed end. The closed end is shaped so as to permit unrestricted entry into a body through a channel thereof and has means associated therewith enabling the opening of the closed end and protrusion therethrough by an inner tube extending within the outer tube upon said inner tube being pushed against the closed end.

WO 97/32517 A1 relates to a protected microbiological sampling brush assembly including a sampling brush mounted on the distal end of a control wire, an inner catheter which houses the brush, an outer catheter which houses the inner catheter, and an elastomeric tip which is rigidly attached to the distal end of the outer catheter.

### Summary

In one aspect of the present invention, a sample collection apparatus comprises: an elongate outer casing having an insertion end and a withdrawal end; and a sample collection device; wherein the apparatus is moveable between an undeployed configuration in which the insertion end is closed and a deployed configuration in which the insertion end is open; wherein the apparatus comprises an opening mechanism which is activatable to open the insertion end of the outer casing as the apparatus moves from the undeployed configuration to the deployed configuration; wherein the opening mechanism comprises an elongate intermediate casing within the outer casing; wherein the elongate outer casing has an insertion end which is closed in the undeployed configuration; wherein the intermediate casing is moveable within the outer casing so that the insertion end of the intermediate casing is able to open the insertion end of the outer casing; and wherein the sample collection device is within the intermediate casing and can be extended through the insertion end of the intermediate casing and the insertion end of the outer casing in the deployed configuration.

The sample collection apparatus is for insertion into a vaginal or anal cavity. The apparatus can be used to collect a sample. In the undeployed configuration the apparatus may be inserted into the cavity. Whilst in the cavity, the apparatus may be deployed or transferred to the deployed configuration in which the collection device can be extended through the open insertion end. The collection device may be withdrawn with the outer casing still in the orifice.

The insertion end of the outer casing may have perforations to assist opening.

The sample collection apparatus may further comprise a stop which limits the extension of the intermediate casing in relation to the outer casing in the deployed configuration.

The insertion end of the intermediate casing may be extendable beyond the insertion end of the outer casing in the deployed configuration.

The intermediate casing may be slideable within the outer casing to move from the undeployed configuration to the deployed configuration.

The intermediate casing may be moveable within the outer casing using a screw thread to move from the undeployed configuration to the deployed configuration.

The insertion end of the intermediate casing may be closed in the undeployed configuration.

The insertion end of the intermediate casing may have perforations to assist opening.

The insertion end of the outer casing may have an inner curvature and the insertion end of the intermediate casing may have an outer curvature, wherein said inner curvature is more curved than said outer curvature.

The intermediate casing may be longer than the outer casing and a withdrawal end of the intermediate casing may extend beyond the withdrawal end of the outer casing in the undeployed configuration.

The sample collection apparatus may comprise a further opening mechanism which is activatable to open the insertion end of the intermediate casing as the apparatus moves from the undeployed configuration to the deployed configuration. The further opening mechanism may comprise an elongate inner casing within the intermediate casing wherein the elongate inner casing has an insertion end and is moveable within the intermediate casing so that the insertion end of the inner casing is able to open the insertion end of the intermediate casing, and wherein the sample collection device is within the inner casing and can be extended through the insertion end of the inner casing, the insertion end of the intermediate casing and the insertion end of the outer casing in the deployed configuration.

The apparatus may comprise a stop which limits the extension of the inner casing in relation to the intermediate casing in the deployed configuration.

The insertion end of the inner casing may be extendable beyond the insertion end of the intermediate casing in the deployed configuration.

The inner casing may be slideable within the intermediate casing to move from the undeployed configuration to the deployed configuration or the inner casing may be moveable within the intermediate casing using a screw thread to move from the undeployed configuration to the deployed configuration.

The inner casing may be longer than the intermediate casing and a withdrawal end of the inner casing may extend beyond the withdrawal end of the intermediate casing in the undeployed configuration.

The sample collection apparatus may comprise a sample collection device, wherein the sample collection device is longer than the outer casing, intermediate casing and/or inner casing and a withdrawal end of the sample collection device extends beyond the withdrawal end of the outer casing, intermediate casing and/or inner casing in the undeployed configuration.

The sample collection device may comprise a swab, a brush or a spatula.

The apparatus may comprise a guide for guiding the sample collection device within the apparatus.

The sample collection apparatus may comprise a guard on the withdrawal end of the outer casing.

The sample collection device may have a detachable head.

Another aspect of the present invention provides a kit comprising the components of a sample collection apparatus.

Embodiments of the invention can provide a smaller tool for insertion into the vaginal or anal cavity to helps limit the level of discomfort endured.

Embodiments of the present invention enable a less invasive technique to be used than current techniques. Also, the apparatus has a protective casing or covering to ensure that the sample collection device (e.g. swab) will take the sample from the target area, without being contaminated while introducing it in or retrieving it from the vaginal canal.Embodiments of the invention use a protective outer casing or shield encasing the collection device (e.g. a swab). Embodiments can provide a mechanism for a staggered exposure which can be used, for example, for sampling different regions of the vaginal canal. This significantly reduces the potential for contamination.

Other aspects and features of the present invention will be appreciated from the following description and the accompanying claims.

### Brief Description of the Drawings

Embodiments of the present invention will now be described, by way of example only, with reference to the accompanying drawings in which like reference numerals are used to depict like parts. In the drawings:
Fig. 1 a shows a first exemplary embodiment of a sample collection apparatus when the apparatus is in an undeployed configuration;
Fig. 1b shows a cross section of a first exemplary embodiment of a sample collection apparatus when the apparatus is in an undeployed configuration;
Fig. 2a shows a first exemplary embodiment of a sample collection apparatus when the apparatus is in a first partially deployed configuration;
Fig. 2b shows a cross section of a first exemplary embodiment of a sample collection apparatus when the apparatus is in a first partially deployed configuration;
Fig. 3a shows a first exemplary embodiment of a sample collection apparatus when the apparatus is in a fully deployed configuration;
Fig. 3b shows a cross section of a first exemplary embodiment of a sample collection apparatus when the apparatus is in a fully deployed configuration;
Fig. 4 shows a cross section of the insertion end of a sample collection apparatus in an undeployed configuration;
Fig. 5 shows a cross section of the insertion end of a sample collection apparatus in a first partially deployed configuration;
Fig. 6 shows a cross section of the insertion end of a sample collection apparatus in a fully deployed configuration;
Fig. 7a shows a cutaway picture of a second exemplary embodiment of the sample collection apparatus, wherein the outer casing is shown as a cross section, and the apparatus is in an undeployed configuration;
Fig. 7b shows a cutaway picture of a second exemplary embodiment of the sample collection apparatus, wherein the outer casing and intermediate casing are shown as a cross section, and the apparatus is in an undeployed configuration;
Fig. 8 shows a cross section of the second exemplary embodiment of the sample collection apparatus in a first partially deployed configuration;
Fig. 9 shows a cross section of the second exemplary embodiment of the sample collection apparatus in a fully deployed configuration;
Fig. 10 shows the second exemplary embodiment of the sample collection apparatus from a perspective view;
Fig. 11 shows the second exemplary embodiment of the sample collection apparatus from a cutaway, perspective view;
Fig. 12 shows the second exemplary embodiment of the sample collection apparatus in a disassembled form;
Fig. 13a shows a cross-section of a third exemplary embodiment of the sample collection apparatus; and
Fig. 13b shows a cutaway, perspective view of the third exemplary embodiment of the sample collection apparatus.

### Detailed Description

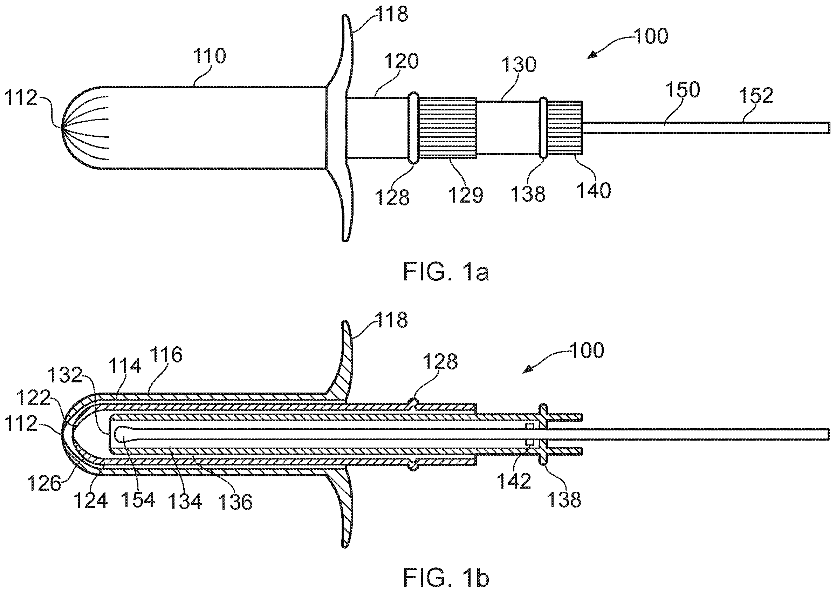

Figures 1a and 1b depict an exemplary embodiment of the sample collection apparatus 100 in an undeployed configuration. In particular, Fig. 1 a depicts the sample collection apparatus 100 from an external viewpoint and Fig. 1b depicts a cross-section of the sample collection apparatus 100. The sample collection apparatus 100 comprises an elongate outer casing 110 with a closed insertion end 112 and inner and outer surfaces 114 and 116 respectively. The closed insertion end 112 is configured to be openable. In this embodiment, the closed insertion end is petalled so that the end may be opened if pressure is applied from within the outer casing 110.

The elongate outer casing may also have a guard 118 fixed on the withdrawal end, arranged to limit the distance that the outer casing 110 may be inserted into an orifice from which a sample is to be taken. The guard may also serve to provide a surface for the stopper 128 to press against in the first partially deployed configuration. The guard may also serve to allow a user to apply pressure to the various casings provided in the sample collection apparatus, in order for the apparatus to be deployed. The guard can further restrict lateral movements of the sample collection apparatus 100 when it is deployed in an orifice.

The sample collection apparatus 100 of this exemplary embodiment further comprises an intermediate casing 120 arranged within the outer casing 110 and capable of telescoping through the outer casing 110. The intermediate casing also has a closed insertion end 122 which in this embodiment is a petalled end configured to open if pressure is applied from within the intermediate casing 120. The intermediate casing has an inner surface 124 and an outer surface 126. The intermediate casing 120 has a stopper 128 arranged on the withdrawal end to limit the distance that the intermediate casing 120 may telescope through the outer casing 110. The intermediate casing 120 also has a grip 129. The elongate intermediate casing 120 is longer than the elongate outer casing 110.

The sample collection apparatus 100 depicted in Figures 1a and 1b further comprises an inner casing 130 arranged inside the intermediate casing 120 and capable of telescoping through the intermediate casing 120. The inner casing 130 has an inner surface 134 and an outer surface 136. The inner casing has an open insertion end 132. The inner casing 130 also has a guide 142 arranged to guide the movement of a sample collection device. The inner casing 130 also has a stopper 138 arranged to limit the distance that the inner casing 130 can move within or telescope through the intermediate casing 120. The inner casing 130 also has a grip 140. The inner casing 130 is longer than the intermediate casing 120 and the outer casing 110.

The sample collection device is a swab 150 comprising a stem 152 and a swab head 154. The swab is arranged inside the inner casing 130 as shown in Fig. 1 b. The swab 150 is longer than the outer casing 110, the intermediate casing 120 and the inner casing 130.

In the undeployed configuration depicted in Figs. 1a and 1b, the petalled insertion end 112 of the outer casing 110 is closed. The petalled insertion end 122 of the intermediate casing 120 is also closed. In this exemplary embodiment the intermediate casing 120 is arranged so that, when the intermediate casing is pushed towards the closed insertion end 112 of the outer casing 110, the outer surface 126 of the intermediate casing 120 comes into contact with the inner surface 114 of the outer casing 110 and opens the petalled insertion end 112 of the outer casing 110. That is, the intermediate casing 120 has the dual role of acting as an opening mechanism for the outer casing 110 and protecting the inner casing 130.

The intermediate casing 120 and the outer casing 110 are shaped so that the tip of the insertion end 122 of the intermediate casing 120 is unable to come into contact with the tip of the insertion end 112 of the outer casing 110. This limits the risk of contamination when the petalled insertion end 112 of the outer casing 110 is opened. The ends of the casings are curved so that the closed insertion end 112 of the outer casing 110 is configured to be opened when the outer surface 126 of the intermediate casing 120 applies pressure to the inner surface 114 of the outer casing 110 at a point other than at the tip.

In the undeployed configuration of the sample collection apparatus 100, the inner casing 130 is arranged within the intermediate casing 120 so that, when the inner casing 130 is pushed towards the closed insertion end 122 of the intermediate casing 120, the outer surface 136 of the inner casing 130 comes into contact with the inner surface 124 of the intermediate casing 120 and opens the petalled insertion end 122 of the intermediate casing 120.

The closed, petalled insertion ends 112 and 122 have perforations to assist in their opening.

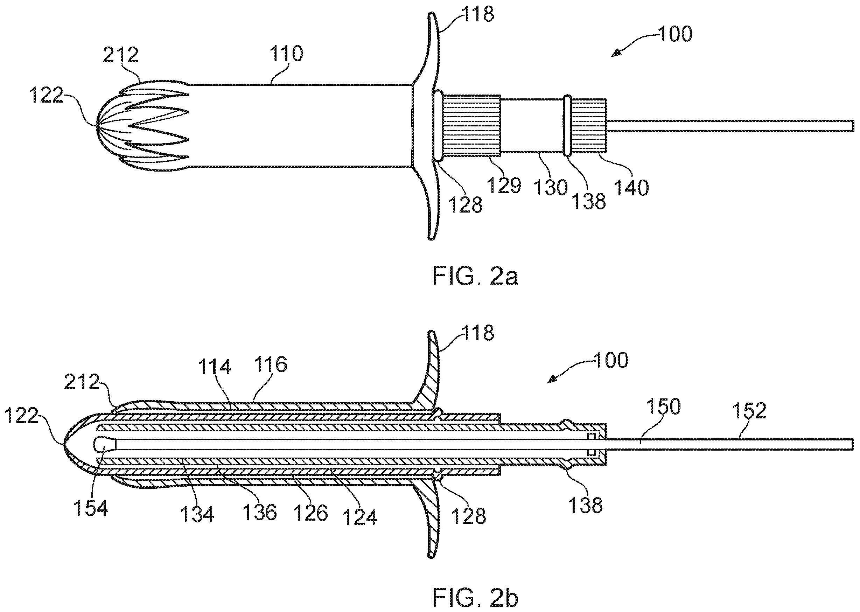

Figs. 2a and 2b depict the same embodiment of the sample collection apparatus 100 in a first partially deployed configuration. More specifically, Fig. 2a shows the sample collection apparatus 100 in the first partially deployed configuration from an external viewpoint and Fig. 2b shows a cross section of the sample collection apparatus in this first partially deployed configuration.

In Figs. 2a and 2b, the intermediate casing 120 is shown extended through the open, petalled end 212 of the outer casing 110. The intermediate casing 120 is depicted as having been slidably moved a limited distance through the open end 212 of the outer casing 110. In this first partial deployment, the guard 118 and the stopper 128 are arranged so as to prevent the intermediate casing 120 telescoping further through the petalled, open end 212 of the outer casing 110.

In order to move from the undeployed configuration to the first partially deployed configuration, a user may, for example, use the grip 129 and push the intermediate casing towards the orifice. The motion causes the intermediate casing 120 to force open the closed insertion end 112 of the outer casing 110. The intermediate casing 120 can be extended until the stopper 128 comes into contact with the guard 118.

The outer surface 126 of the intermediate casing 120 is not in contact with the outer surface 116 of the outer casing 110 in this partially deployed configuration. This means that cells or semen from, for example, the lower vaginal region should not be transferred on the intermediate casing and thus should not be transferred to a higher region of the vagina. Thus, the risk of contamination of a sample collection from the higher vaginal region is reduced as cells or semen from the lower vaginal region should not be transferred to the higher vaginal region.

The petalled insertion end 122 of the intermediate casing 120 is closed and so shields the inner casing 130 and the sample collection device 150.

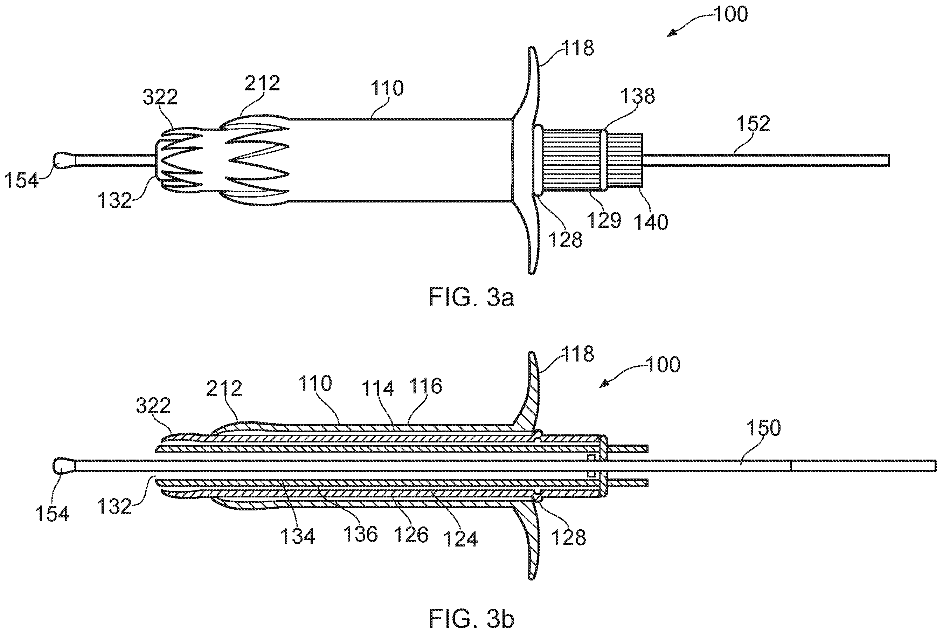

Figs. 3a and 3b depict the same embodiment of the sample collection apparatus in a fully deployed configuration. Fig 3a depicts an external view of the apparatus whilst Fig 3b shows a cross section of the sample collection apparatus.

In this fully deployed configuration, the inner casing 130 is extended through the open petalled end 212 of the outer casing 110 and the open petalled end 322 of the intermediate casing 120. The stopper 138 of the inner casing 130 is in contact with the grip 129 of the intermediate casing 120, thereby inhibiting further progression of the inner casing 130 through the intermediate casing 120.

The swab 150 is extended by the user through the inner casing 130 so that the swab head 154 is exposed, and thus suitably arranged to collect cells or other samples.

To move from the first partially deployed configuration shown in Figs. 2a and 2b to the fully deployed configuration of the apparatus 100 shown in Figs. 3a and 3b, the user can use the grip 140 to slide the inner casing 130 through the intermediate casing 120 until the end of the intermediate casing 120 is penetrated. Thus the apparatus 100 adopts a second partially deployed configuration (not shown) wherein the petalled insertion ends of the outer casing and intermediate casing have both been opened and yet the sample collection device remains hidden away, protected by the inner casing 130. After the insertion end 212 of the intermediate casing 120 is opened, the user may optionally extend the inner casing 130 further through the intermediate casing as desired, but no further than the stopper 138 allows.

The inner casing 130 acts as a protector for the swab 150 and prevents exposure of the swab head 154 to contaminants on the extended intermediate casing 120, particularly when the insertion end of the intermediate casing is being opened. To progress from the second partially deployed configuration to the fully deployed configuration shown in Figs. 3a and 3b, a user may slide the swab 150 along the inner casing until the swab head 154 is exposed.

If, for example, the sample collection apparatus 100 is used for a sexual examination, then in the fully deployed configuration the sample collection device would collect a sample from the higher vaginal region with a very low risk of contamination from material carried from the lower vaginal region.

In this embodiment, the swab head 154 can be retracted within the apparatus after a sample has been collected. The apparatus can then be withdrawn and deposited in, for example, an evidence bag.

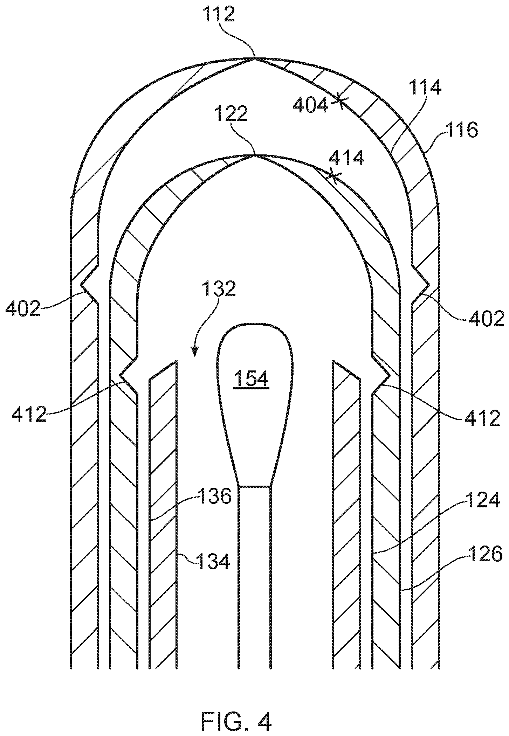

Figure 4 depicts the insertion end of the same embodiment of the sample collection apparatus arranged in the undeployed configuration. The swab 150 is arranged inside the inner casing 130, which in turn is arranged inside the intermediate casing 120. The intermediate casing 120 is arranged inside the outer casing 110.

The petalled insertion end 112 of the outer casing 110 is closed. The petalled insertion end 122 of the intermediate casing 120 is also closed. The closed insertion ends of the outer and intermediate casings comprise perforations to assist the opening of the insertion ends. The inner surface 114 of the outer casing 110 has indents 402 configured to ease the opening of the petalled insertion end 112. Similarly, the inner surface 124 of the intermediate casing 120 has indents 412 configured to assist in the opening of the petalled insertion end 122 of the intermediate casing 120.

In this embodiment, the closed insertion end 122 of the intermediate casing 120 is curved differently to the closed insertion end 112 of the outer casing 110. This difference in curvature prevents the tips of the outer and intermediate casings from touching. The closed insertion end of the intermediate casing is instead configured so that a point 414 on the outer surface 126, not at the tip, makes contact with a point 404 on the inner surface 114, not at the tip, in order to apply pressure to open the insertion end 112 of the outer casing 110.

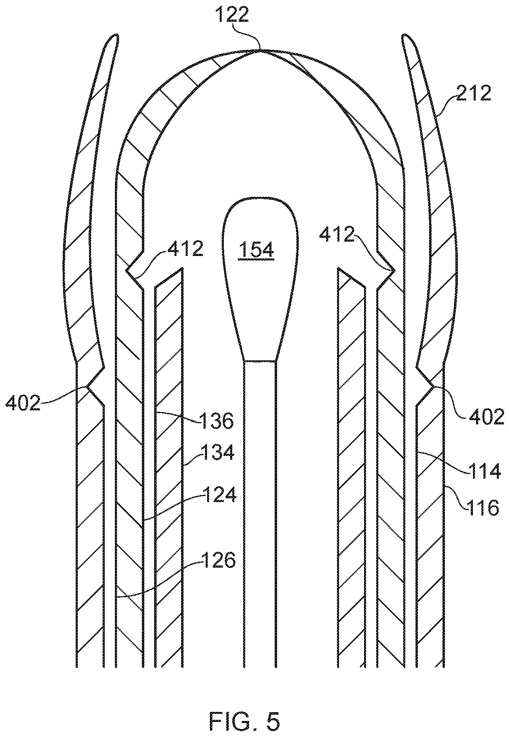

Fig. 5 depicts the insertion end of the sample collection apparatus arranged in the first partially deployed configuration. The petalled end 212 of the outer casing is open, exposing the intermediate casing 120. As has been described previously, the curvature of the outer casing 110 and the shaping of the intermediate casing 120 prevent the tip of the closed insertion end 122 of the intermediate casing from coming into contact with the tip of the outer casing 110.

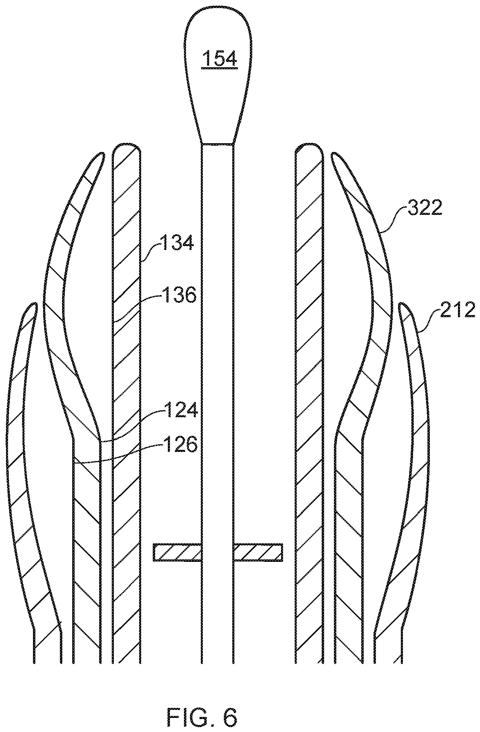

Fig. 6 depicts the insertion end of the fully deployed sample collection apparatus. In this configuration, both the end of the outer casing 110 and the end of the intermediate casing 120 are opened and the inner casing 130 is extended. The swab 150 has been extended beyond the end of the inner casing 130 so that the swab head 154 is exposed.

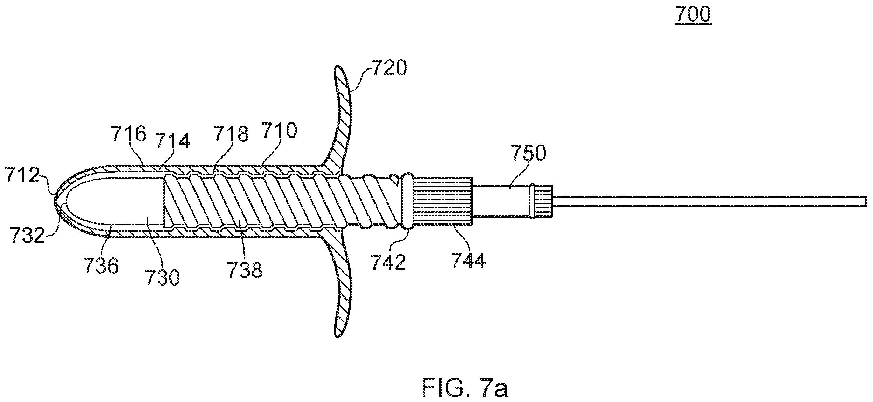

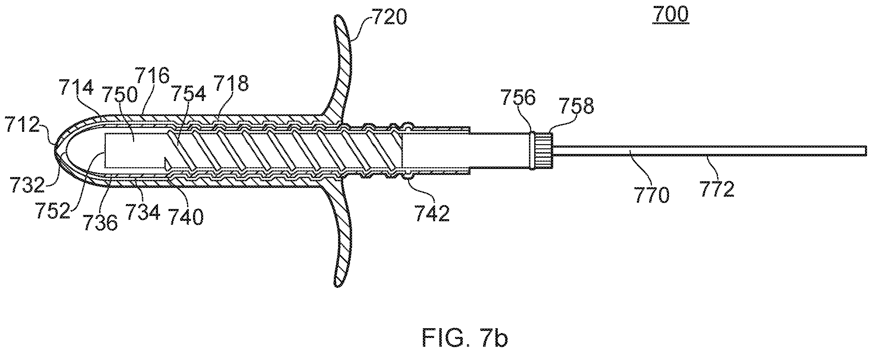

Figs 7a and 7b depict a second embodiment of the sample collection apparatus 700 in an undeployed configuration. Fig. 7a is a cutaway drawing of the sample collection apparatus 700 in which the outer casing 710 is shown in cross section. Fig. 7b is another cutaway drawing of the sample collection apparatus 700 in which the outer casing 710 and the intermediate casing 730 are shown in cross section only.

The outer casing 710 has a closed insertion end 712 and outer surface 716. The outer casing 710 further has an inner surface 714 having an internal thread 718. The outer casing also a guard 720 on the withdrawal end.

The intermediate casing 730 also has a closed insertion end 732 and an outer surface 736 having an external thread 738. The external thread 738 of the intermediate casing 730 is designed to fit the internal thread 718 of the outer casing 710. The inner surface 734 of the intermediate casing 730 has an internal thread 740. The intermediate casing 730 has a stopper 742 arranged to stop the intermediate casing 730 from sliding too far through the outer casing 710. The intermediate casing 730 also has a grip 744. The intermediate casing 730 is longer than the outer casing 710.

An inner casing 750 has an open end 752. The outer surface of the inner casing 750 has an external thread 754, designed to couple with the internal thread of the intermediate casing 730. The inner casing 750 also has a guide (not shown) arranged to guide the motion of a sample collection device 770 through the inner casing 750. The inner casing 750 also has a stopper 756 arranged to limit the distance that the inner casing 750 can travel through the intermediate casing 730.

The inner casing 750 also has a grip 758. The inner casing 750 is longer than the intermediate casing 730 and the outer casing 710.

In this embodiment, the sample collection apparatus is configured to be deployable by screwing the inner and intermediate casing into the outer casing 710 and opening the ends 712 and 732 of the outer casing 710 and intermediate casing 730 respectively. In the undeployed configuration, the petalled insertion ends of the outer casing and intermediate casing are closed.

As with the first embodiment, the curvature of the insertion end of the outer casing is different from the curvature of the insertion end of the intermediate casing, so that the intermediate casing can be used to open the insertion end of the outer casing without the tip of the insertion end of the intermediate casing coming into contact with the tip of the insertion end of the outer casing.

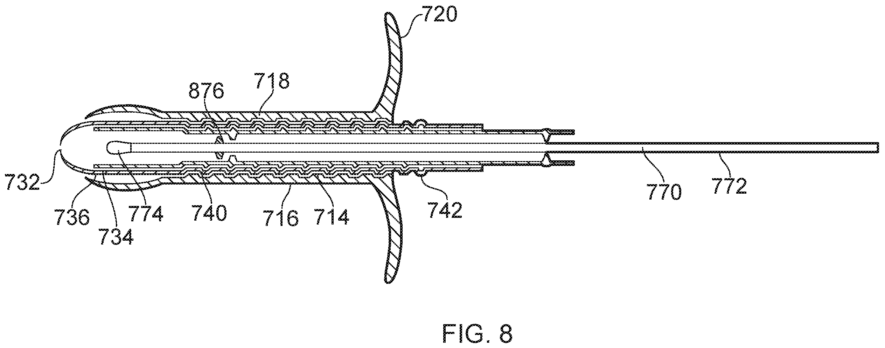

Fig. 8 depicts a cross sectional representation of the second embodiment of the sample collection apparatus 700 in a first partially deployed configuration. In this configuration, the intermediate casing 730 is extended through the open end of the outer casing 710 but the insertion end 732 of the intermediate casing is closed. The distance that the intermediate casing can extend through the outer casing is limited by the stopper 738.

As can also be seen from Fig. 8, the sample collection device 770 is arranged to be slidable within the inner casing 750, and is guided by a guide 876. The sample collection device 770 is a swab comprising a stem 772 and a swab head 774.

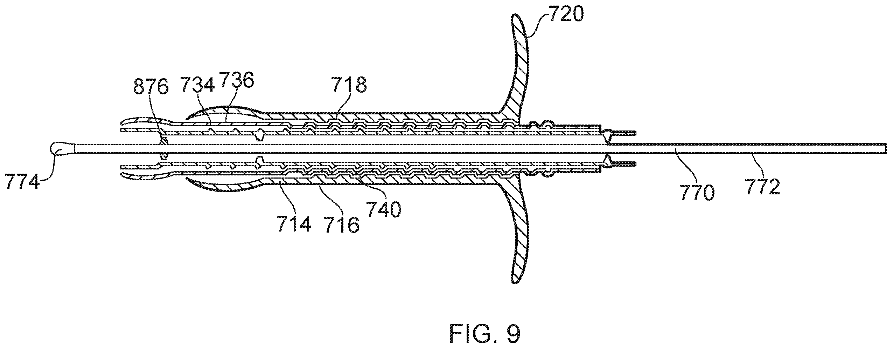

Fig. 9 is a cross sectional diagram of the sample collection apparatus 700 in the fully deployed configuration. In the fully deployed configuration, the ends of the outer casing and the intermediate casing are both open. The inner casing 750 is extended through the intermediate casing 730 by a distance limited by the stopper 756. The swab head 774 is exposed for the collection of a sample.

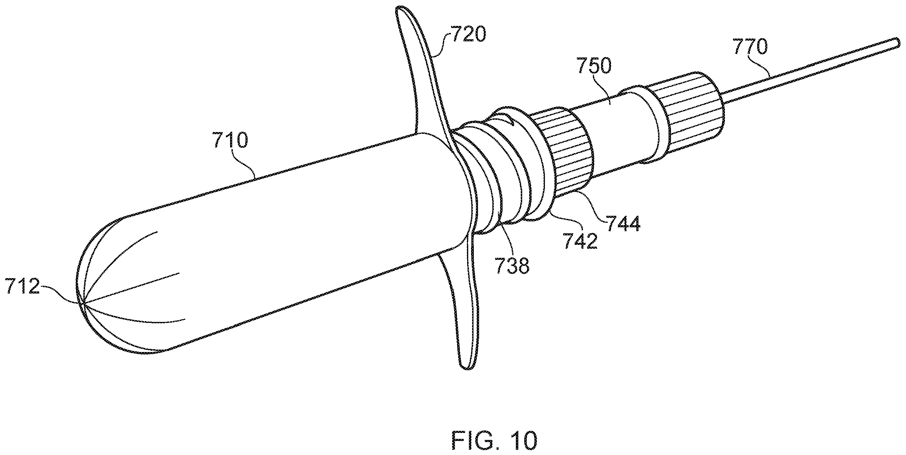

Fig. 10 offers is a perspective view of the second embodiment of the sample collection apparatus when the sample collection apparatus is in an undeployed configuration.

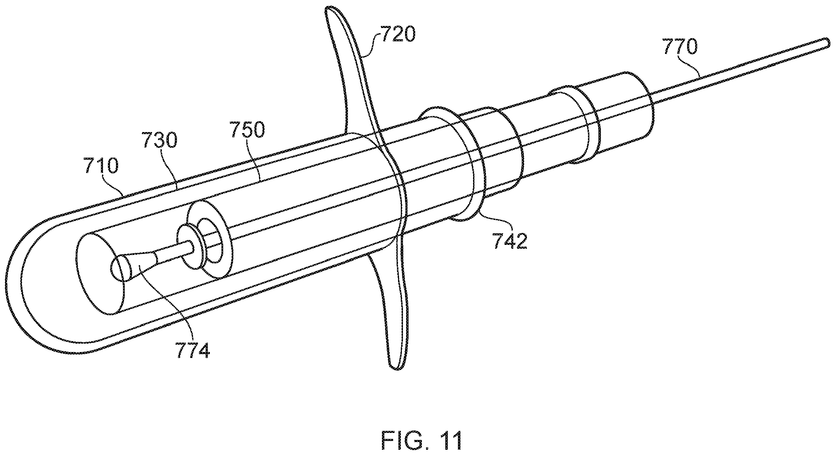

Fig. 11 is a perspective, cutaway picture of the sample collection apparatus when the sample collection apparatus is in an undeployed configuration. The insertion ends of the outer casing and intermediate casing are closed.

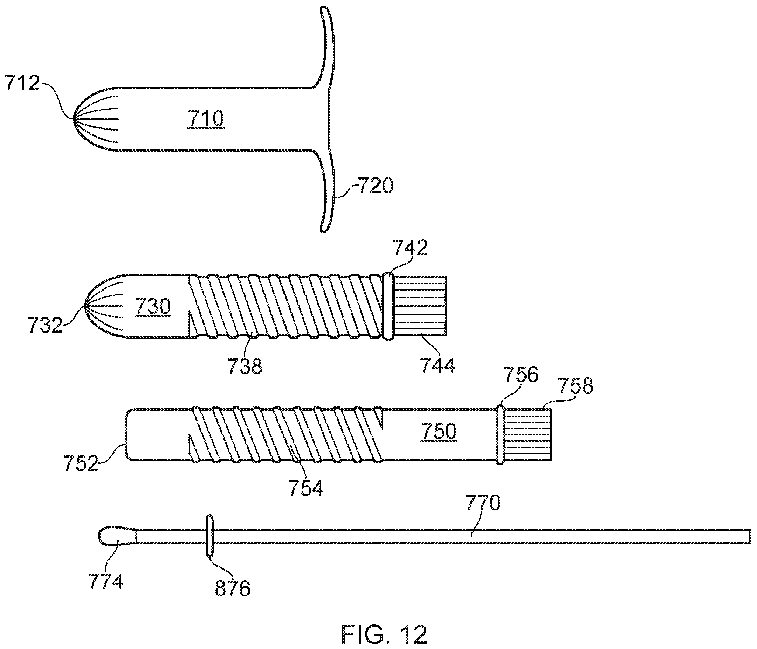

Fig. 12 shows a disassembled sample collection apparatus according to the second embodiment.

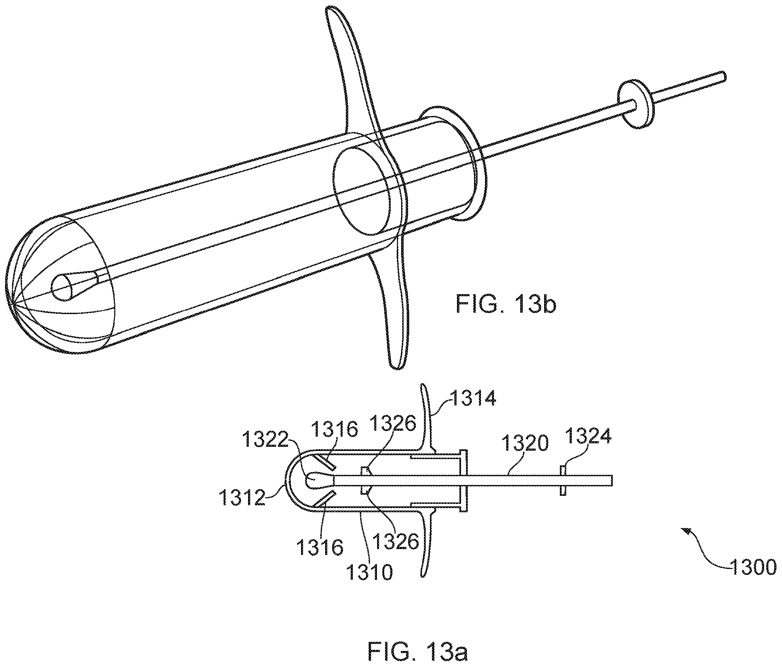

Figs. 13a and 13b show a third embodiment of the sample collection apparatus 1300. In this embodiment, the sample collection apparatus 1300 comprises an outer casing 1310, an opening mechanism, and a sample collection device 1320. Fig. 13a depicts a cross section of the third embodiment of the sample collection apparatus when it is in an undeployed configuration. Fig. 13b shows the sample collection apparatus 1300 from a perspective view.

The outer casing 1310 has a closed insertion end 1312 that is configured to be openable by a catch 1316. The outer casing 1310 also has a guard 1314 arranged to prevent the sample collection apparatus 1300 from being inserted too far into the cavity.

The sample collection device 1320 has a head 1322 suitable for collecting a sample when exposed, a stopper 1324 suitable for limiting the distance that the sample collection device 1320 can be moved, and one or more protrusions 1326 configured to connect with the catches 1326.

As the sample collection device 1320 is moved towards the closed insertion end of the outer casing 1310, the protrusions 1326 on the sample collection device connect with the catch mechanism 1316 on the outer casing, forcing the insertion end 1312 to open. The sample collection device 1320 can then be further moved into the cavity, beyond the end 1312 of the outer casing 1310, thus exposing the head 1322 of the sample collection device 1320, but no further than the stopper 1324 allows.

The sample collection apparatus may be manufactured from any suitable material. For example, the sample collection apparatus may be made from plastic. Alternatively, the sample collection apparatus may be made from waxed cardboard.

Variations of the described embodiments are envisaged, for example, the features of all of the disclosed embodiments may be combined in any way.

In a simple embodiment the apparatus may comprise an elongate outer casing and a sample collection device which can itself be used to open the closed insertion end of the casing.

Other variations of the described embodiments are also contemplated. For example, the sample collection device may comprise a swab. The sample collection device may comprise a spatula or brush. The sample collection device may have a detachable head.

More or fewer casings may be used. The intermediate and inner casings may not be necessary, and the sample collection apparatus may be operable with an outer casing and a sample collection device, wherein some other means is used to open the sealed end of the outer casing. Alternatively, there may be, for example, a third casing arranged between the inner casing and the sample collection device.

The sample collection device may be retractable, and so may be withdrawn within the sample collection apparatus. The sample collection apparatus may then be withdrawn and deposited in, for example, an evidence bag.

The inner casing may be removable from the apparatus. The sample collection device can be retracted inside the inner casing and then the sample collection device and inner casing can be removed from the apparatus whilst leaving the outer casing and intermediate casing in place. The sample collection device can then be removed from the inner casing and the sample collection device or the detachable head of the device can be deposited in, for example, a microcentrifuge tube.

One or more different opening mechanisms may be used. For example, the insertion ends of the outer casing and intermediate casing may be closed with a penetrable seal or membrane. The apparatus may then be configured such that the intermediate casing is able to pierce the penetrable seal of the outer casing and the inner casing is able to pierce the penetrable seal of the intermediate casing. Alternatively some other catch mechanism can be used for example.

One or more guides may be used to guide the sample collection device as the apparatus moves from an undeployed configuration to a deployed configuration.

A guard may not be necessary. However, if a guard is present on the withdrawal end of the outer casing, it may be circular or may comprise one or more protrusions.

A camera may be configured to be used with the sample collection apparatus for visualisation. Such a camera may, for example, be fitted within the outer casing of the apparatus and may be used to assist in guiding the sample collection device.

In the second embodiment the external threads are fitted to the outer surfaces of the intermediate and inner casings and the internal threads are fitted to the inner surfaces of the outer casing and the intermediate casing. The internal threads can be fitted to the outer surfaces of the intermediate and inner casings and the external threads can be fitted to the inner surfaces of the outer casing and the intermediate casing. A screw mechanism may, for example, be used for manoeuvring the intermediate casing through the outer casing whilst a push mechanism, such as that used in the first embodiment may be used to move the inner casing through the intermediate casing. A combination of mechanisms may be used, such as the sliding mechanism of the first embodiment and the screw mechanism of the second embodiment, to deploy the sample collection apparatus.

The above embodiments have been described by way of example only, and the described embodiments are to be considered in all respects only as illustrative and not restrictive. It will be appreciated that variations of the described embodiments may be made without departing from the scope of the invention which is indicated by the appended claims rather than by the foregoing description.

## Claims

1. A sample collection apparatus (100) comprising:
an elongate outer casing (110) having an insertion end (112) and a withdrawal end; and
a sample collection device;
wherein the apparatus is moveable between an undeployed configuration in which the insertion end is closed and a deployed configuration in which the insertion end is open;
wherein the apparatus comprises an opening mechanism which is activatable to open the insertion end of the outer casing as the apparatus moves from the undeployed configuration to the deployed configuration;
wherein the opening mechanism comprises an elongate intermediate casing (120) within the outer casing;
wherein the elongate intermediate casing has an insertion end (122) which is closed in the undeployed configuration;
wherein the intermediate casing is moveable within the outer casing so that the insertion end of the intermediate casing is able to open the insertion end of the outer casing;
and wherein the sample collection device is within the intermediate casing and can be extended through the insertion end of the intermediate casing and the insertion end of the outer casing in the deployed configuration.

2. A sample collection apparatus according to claim 1, wherein the insertion end of the outer casing has perforations to assist opening.

3. A sample collection apparatus according to claim 1 or claim 2, wherein the apparatus comprises a stop (128) which limits the extension of the intermediate casing in relation to the outer casing in the deployed configuration.

4. A sample collection apparatus according to any one of claims 1 to 3, wherein the insertion end of the intermediate casing is extendable beyond the insertion end of the outer casing in the deployed configuration.

5. A sample collection apparatus according to any one of claims 1 to 4, wherein the intermediate casing is slideable within the outer casing to move from the undeployed configuration to the deployed configuration or wherein the intermediate casing is moveable within the outer casing using a screw thread to move from the undeployed configuration to the deployed configuration.

6. A sample collection apparatus according to any of claims 1 to 5, wherein the insertion end of the intermediate casing has perforations to assist opening.

7. A sample collection apparatus according to any of claims 1 to 6, wherein the insertion end of the outer casing has an inner curvature and the insertion end of the intermediate casing has an outer curvature, wherein said inner curvature is more curved than said outer curvature.

8. A sample collection apparatus according to any of claims 1 to 7, wherein the intermediate casing is longer than the outer casing and a withdrawal end of the intermediate casing extends beyond the withdrawal end of the outer casing in the undeployed configuration.

9. A sample collection apparatus according to any of claims 1 to 8, wherein the apparatus comprises a further opening mechanism which is activatable to open the insertion end of the intermediate casing as the apparatus moves from the undeployed configuration to the deployed configuration.

10. A sample collection apparatus according to claim 9, the further opening mechanism comprising an elongate inner casing (130) within the intermediate casing wherein the elongate inner casing has an insertion end (132) and is moveable within the intermediate casing so that the insertion end of the inner casing is able to open the insertion end of the intermediate casing, and wherein the sample collection device is within the inner casing and can be extended through the insertion end of the inner casing, the insertion end of the intermediate casing and the insertion end of the outer casing in the deployed configuration.

11. A sample collection apparatus according to claim 10, wherein the apparatus comprises a stop (138) which limits the extension of the inner casing in relation to the intermediate casing in the deployed configuration; and/or wherein the insertion end of the inner casing is extendable beyond the insertion end of the intermediate casing in the deployed configuration.

12. A sample collection apparatus according to claim 10 or claim 11, wherein the inner casing is slideable within the intermediate casing to move from the undeployed configuration to the deployed configuration or wherein the inner casing is moveable within the intermediate casing using a screw thread to move from the undeployed configuration to the deployed configuration; or wherein the inner casing is longer than the intermediate casing and a withdrawal end of the inner casing extends beyond the withdrawal end of the intermediate casing in the undeployed configuration.

13. A sample collection apparatus according to any preceding claim, wherein the sample collection device is longer than the outer casing, intermediate casing and/or inner casing and a withdrawal end of the sample collection device extends beyond the withdrawal end of the outer casing, intermediate casing and/or inner casing in the undeployed configuration.

14. A sample collection apparatus according to any preceding claim, wherein the sample collection device comprises a swab (150); and/or wherein the sample collection device comprises a spatula or brush; and/or wherein the apparatus comprises a guide for guiding the sample collection device within the apparatus and/or a guard (118) on the withdrawal end of the outer casing; and/or wherein the sample collection device has a detachable head; and/or wherein the sample collection apparatus is configured to be used with a camera; and/or wherein the sample collection apparatus has a camera fitted within the outer casing.

15. A kit comprising the components of a sample collection apparatus according to any preceding claim.

## Patentansprüche

1. Probenahmevorrichtung (100), aufweisend:
ein längliches äußeres Gehäuse (110) mit einem Einführungsende (112) und einem Entnahmeende; und
eine Probenahmeeinrichtung;
wobei die Vorrichtung zwischen einer Nichteinsatzkonfiguration, in der das Einführungsende geschlossen ist, und einer Einsatzkonfiguration, in der das Einführungsende offen ist, bewegbar ist;
wobei die Vorrichtung einen Öffnungsmechanismus aufweist, der aktivierbar ist, um das Einführungsende des äußeren Gehäuses zu öffnen, während sich die Vorrichtung aus der Nichteinsatzkonfiguration in die Einsatzkonfiguration bewegt;
wobei der Öffnungsmechanismus ein längliches Zwischengehäuse (120) innerhalb des äußeren Gehäuses aufweist;
wobei das längliche Zwischengehäuse ein Einführungsende (122) hat, das in der Nichteinsatzkonfiguration geschlossen ist;
wobei das Zwischengehäuse innerhalb des äußeren Gehäuses bewegbar ist, sodass das Einführungsende des Zwischengehäuses imstande ist, das Einführungsende des äußeren Gehäuses zu öffnen;
und wobei sich die Probenahmeeinrichtung innerhalb des Zwischengehäuses befindet und in der Einsatzkonfiguration durch das Einführungsende des Zwischengehäuses und das Einführungsende des äußeren Gehäuses ausgerückt werden kann.

2. Probenahmevorrichtung nach Anspruch 1, wobei das Einführungsende des äußeren Gehäuses Perforationen hat, die ein Öffnen unterstützen.

3. Probenahmevorrichtung nach Anspruch 1 oder Anspruch 2, wobei die Vorrichtung einen Anschlag (128) aufweist, der das Ausrücken des Zwischengehäuses in Bezug auf das äußere Gehäuse in der Einsatzkonfiguration begrenzt.

4. Probenahmevorrichtung nach einem der Ansprüche 1 bis 3, wobei das Einführungsende des Zwischengehäuses in der Einsatzkonfiguration über das Einführungsende des äußeren Gehäuses hinaus ausrückbar ist.

5. Probenahmevorrichtung nach einem der Ansprüche 1 bis 4, wobei das Zwischengehäuse innerhalb des äußeren Gehäuses gleiten kann, um sich aus der Nichteinsatzkonfiguration in die Einsatzkonfiguration zu bewegen, oder wobei das Zwischengehäuse innerhalb des äußeren Gehäuses mithilfe eines Schraubengewindes bewegbar ist, um sich aus der Nichteinsatzkonfiguration in die Einsatzkonfiguration zu bewegen.

6. Probenahmevorrichtung nach einem der Ansprüche 1 bis 5, wobei das Einführungsende des Zwischengehäuses Perforationen hat, die ein Öffnen unterstützen.

7. Probenahmevorrichtung nach einem der Ansprüche 1 bis 6, wobei das Einführungsende des äußeren Gehäuses eine innere Krümmung hat und das Einführungsende des Zwischengehäuses eine äußere Krümmung hat, wobei die innere Krümmung stärker gekrümmt ist als die äußere Krümmung.

8. Probenahmevorrichtung nach einem der Ansprüche 1 bis 7, wobei das Zwischengehäuse länger als das äußere Gehäuse ist und ein Entnahmeende des Zwischengehäuses sich in der Nichteinsatzposition über das Entnahmeende des äußeren Gehäuses hinaus erstreckt.

9. Probenahmevorrichtung nach einem der Ansprüche 1 bis 8, wobei die Vorrichtung einen weiteren Öffnungsmechanismus aufweist, der aktivierbar ist, um das Einführungsende des Zwischengehäuses zu öffnen, während sich die Vorrichtung aus der Nichteinsatzkonfiguration in die Einsatzkonfiguration bewegt.

10. Probenahmevorrichtung nach Anspruch 9, wobei der weitere Öffnungsmechanismus ein längliches inneres Gehäuse (130) innerhalb des Zwischengehäuses aufweist, wobei das längliche innere Gehäuse ein Einführungsende (132) hat und innerhalb des Zwischengehäuses bewegbar ist, sodass das Einführungsende des inneren Gehäuses das Einführungsende des Zwischengehäuses öffnen kann, und wobei sich die Probenahmeeinrichtung innerhalb des inneren Gehäuses befindet und in der Einsatzkonfiguration durch das Einführungsende des inneren Gehäuses, das Einführungsende des Zwischengehäuses und das Einführungsende des äußeren Gehäuses ausgerückt werden kann.

11. Probenahmevorrichtung nach Anspruch 10, wobei die Vorrichtung einen Anschlag (138) aufweist, der das Ausrücken des inneren Gehäuses in Bezug auf das Zwischengehäuse in der Einsatzkonfiguration begrenzt; und/oder wobei das Einführungsende des inneren Gehäuses in der Einsatzkonfiguration über das Einführungsende des Zwischengehäuses hinaus ausrückbar ist.

12. Probenahmevorrichtung nach Anspruch 10 oder Anspruch 11, wobei das innere Gehäuse innerhalb des Zwischengehäuses gleiten kann, um sich aus der Nichteinsatzkonfiguration in die Einsatzkonfiguration zu bewegen, oder wobei das innere Gehäuse innerhalb des Zwischengehäuses mithilfe eines Schraubengewindes bewegbar ist, um sich aus der Nichteinsatzkonfiguration in die Einsatzkonfiguration zu bewegen; oder wobei das innere Gehäuse länger als das Zwischengehäuse ist und ein Entnahmeende des inneren Gehäuses sich in der Nichteinsatzposition über das Entnahmeende des Zwischengehäuses hinaus erstreckt.

13. Probenahmevorrichtung nach einem vorangehenden Anspruch, wobei die Probenahmeeinrichtung länger als das äußere Gehäuse, das Zwischengehäuse und/oder das innere Gehäuse ist und ein Entnahmeende der Probenahmeeinrichtung sich in der Nichteinsatzposition über das Entnahmeende des äußeren Gehäuses, des Zwischengehäuses und/oder des inneren Gehäuses hinaus erstreckt.

14. Probenahmevorrichtung nach einem vorangehenden Anspruch, wobei die Probenahmeeinrichtung einen Tupfer (150) aufweist; und/oder wobei die Probenahmeeinrichtung einen Spatel oder eine Bürste aufweist; und/oder wobei die Vorrichtung eine Führung zum Führen der Probenahmeeinrichtung innerhalb der Vorrichtung und/oder eine Schutzeinrichtung (118) am Entnahmeende des äußeren Gehäuses aufweist; und/oder wobei die Probenahmeeinrichtung einen abnehmbaren Kopf hat; und/oder wobei die Probenahmevorrichtung gestaltet ist, mit einer Kamera verwendet zu werden; und/oder wobei die Probenahmevorrichtung eine Kamera hat, die innerhalb des äußeren Gehäuses angebracht ist.

15. Kit, aufweisend die Komponenten einer Probenahmevorrichtung nach einem vorangehenden Anspruch.

## Revendications

1. Appareil de collecte d'échantillon (100), comprenant:
une enceinte extérieure allongée (110) présentant une extrémité d'insertion (112) et une extrémité de retrait; et
un dispositif de collecte d'échantillon;
dans lequel l'appareil est mobile entre une configuration non déployée, dans laquelle l'extrémité d'insertion est fermée, et une configuration déployée, dans laquelle l'extrémité d'insertion est ouverte;
dans lequel l'appareil comprend un mécanisme d'ouverture qui est activable pour ouvrir l'extrémité d'insertion de l'enceinte extérieure lorsque l'appareil se déplace à partir de la configuration non déployée jusqu'à la configuration déployée;
dans lequel le mécanisme d'ouverture comprend une enceinte intermédiaire allongée (120) à l'intérieur de l'enceinte extérieure;
dans lequel l'enceinte intermédiaire allongée présente une extrémité d'insertion (122) qui est fermée dans la configuration non déployée;
dans lequel l'enceinte intermédiaire est mobile à l'intérieur de l'enceinte extérieure de telle sorte que l'extrémité d'insertion de l'enceinte intermédiaire soit capable d'ouvrir l'extrémité d'insertion de l'enceinte extérieure; et
dans lequel le dispositif de collecte d'échantillon est situé à l'intérieur de l'enceinte intermédiaire et peut être étendu à travers l'extrémité d'insertion de l'enceinte intermédiaire et l'extrémité d'insertion de l'enceinte extérieure dans la configuration déployée.

2. Appareil de collecte d'échantillon selon la revendication 1, dans lequel l'extrémité d'insertion de l'enceinte extérieure comporte des perforations destinées à faciliter l'ouverture.

3. Appareil de collecte d'échantillon selon la revendication 1 ou la revendication 2, dans lequel l'appareil comprend un arrêt (128) qui limite l'extension de l'enceinte intermédiaire par rapport à l'enceinte extérieure dans la configuration déployée.

4. Appareil de collecte d'échantillon selon l'une quelconque des revendications 1 à 3, dans lequel l'extrémité d'insertion de l'enceinte intermédiaire est extensible au-delà de l'extrémité d'insertion de l'enceinte extérieure dans la configuration déployée.

5. Appareil de collecte d'échantillon selon l'une quelconque des revendications 1 à 4, dans lequel l'enceinte intermédiaire est coulissante à l'intérieur de l'enceinte extérieure de manière à se déplacer à partir de la configuration non déployée jusqu'à la configuration déployée, ou dans lequel l'enceinte intermédiaire est mobile à l'intérieur de l'enceinte extérieure en utilisant un filet de vis de manière à se déplacer à partir de la configuration non déployée jusqu'à la configuration déployée.

6. Appareil de collecte d'échantillon selon l'une quelconque des revendications 1 à 5, dans lequel l'extrémité d'insertion de l'enceinte intermédiaire comporte des perforations destinées à faciliter l'ouverture.

7. Appareil de collecte d'échantillon selon l'une quelconque des revendications 1 à 6, dans lequel l'extrémité d'insertion de l'enceinte extérieure présente une courbure intérieure et l'extrémité d'insertion de l'enceinte intermédiaire présente une courbure extérieure, dans lequel ladite courbure intérieure est plus incurvée que ladite courbure extérieure.

8. Appareil de collecte d'échantillon selon l'une quelconque des revendications 1 à 7, dans lequel l'enceinte intermédiaire est plus longue que l'enceinte extérieure et une extrémité de retrait de l'enceinte intermédiaire s'étend au-delà de l'extrémité de retrait de l'enceinte extérieure dans la configuration non déployée.

9. Appareil de collecte d'échantillon selon l'une quelconque des revendications 1 à 8, dans lequel l'appareil comprend un mécanisme d'ouverture supplémentaire qui est activable pour ouvrir l'extrémité d'insertion de l'enceinte intermédiaire lorsque l'appareil se déplace à partir de la configuration non déployée jusqu'à la configuration déployée.

10. Appareil de collecte d'échantillon selon la revendication 9, dans lequel le mécanisme d'ouverture supplémentaire comprend une enceinte intérieure allongée (130) à l'intérieur de l'enceinte intermédiaire, dans lequel l'enceinte intérieure allongée présente une extrémité d'insertion (132) et est mobile à l'intérieur de l'enceinte intermédiaire de telle sorte que l'extrémité d'insertion de l'enceinte intérieure soit capable d'ouvrir l'extrémité d'insertion de l'enceinte intermédiaire, et dans lequel le dispositif de collecte d'échantillon est situé à l'intérieur de l'enceinte intérieure et peut être étendu à travers l'extrémité d'insertion de l'enceinte intérieure, l'extrémité d'insertion de l'enceinte intermédiaire et l'extrémité d'insertion de l'enceinte extérieure dans la configuration déployée.

11. Appareil de collecte d'échantillon selon la revendication 10, dans lequel l'appareil comprend un arrêt (138) qui limite l'extension de l'enceinte intérieure par rapport à l'enceinte intermédiaire dans la configuration déployée et/ou dans lequel l'extrémité d'insertion de l'enceinte intérieure est extensible au-delà de l'extrémité d'insertion de l'enceinte intermédiaire dans la configuration déployée.

12. Appareil de collecte d'échantillon selon la revendication 10 ou la revendication 11, dans lequel l'enceinte intérieure est coulissante à l'intérieur de l'enceinte intermédiaire de manière à se déplacer à partir de la configuration non déployée jusqu'à la configuration déployée, ou dans lequel l'enceinte intérieure est mobile à l'intérieur de l'enceinte intermédiaire en utilisant un filet de vis de manière à se déplacer à partir de la configuration non déployée jusqu'à la configuration déployée; ou dans lequel l'enceinte intérieure est plus longue que l'enceinte intermédiaire et une extrémité de retrait de l'enceinte intérieure s'étend au-delà de l'extrémité de retrait de l'enceinte intermédiaire dans la configuration non déployée.

13. Appareil de collecte d'échantillon selon l'une quelconque des revendications précédentes, dans lequel le dispositif de collecte d'échantillon est plus long que l'enceinte extérieure, l'enceinte intermédiaire et/ou l'enceinte intérieure, et une extrémité de retrait du dispositif de collecte d'échantillon s'étend au-delà de l'extrémité de retrait de l'enceinte extérieure, de l'enceinte intermédiaire et/ou de l'enceinte intérieure dans la configuration non déployée.

14. Appareil de collecte d'échantillon selon l'une quelconque des revendications précédentes, dans lequel le dispositif de collecte d'échantillon comprend un écouvillon (150); et/ou dans lequel le dispositif de collecte d'échantillon comprend une spatule ou une brosse; et/ou dans lequel l'appareil comprend un guide pour guider le dispositif de collecte d'échantillon à l'intérieur de l'appareil et/ou une garde (118) sur l'extrémité de retrait de l'enceinte extérieure; et/ou dans lequel le dispositif de collecte d'échantillon présente une tête détachable; et/ou dans lequel l'appareil de collecte d'échantillon est configuré de manière à être utilisé avec une caméra; et/ou dans lequel l'appareil de collecte d'échantillon comprend une caméra agencée à l'intérieur de l'enceinte extérieure.

15. Kit comprenant les composants d'un appareil de collecte d'échantillon selon l'une quelconque des revendications précédentes.
